# EUROPÄISCHE PATENTANMELDUNG

(11) **EP 0 564 958 A1**
(43) Veröffentlichungstag der Anmeldung: **13.10.1993**
(21) Anmeldenummer: 93105226.0
(22) Anmeldetag: 30.03.1993
(51) Int. Cl.: C12N 7/06, G01N 33/53, G01N 33/569

(54) **Viruslösung zum Einsatz bei Immunoassays**

(30) Priorität: 03.04.1992 DE 4211108
(71) Anmelder: BOEHRINGER MANNHEIM GMBH, D-68298 Mannheim (DE)
(72) Erfinder: Vonwirth, Heiner, Dr., W-7326 Heiningen (DE); Marschall, Michael, Dr., W-8000 München 71 (DE); Bayer, Hubert, Dr., W-8120 Weilheim (DE)

(57) **Zusammenfassung**

Die Erfindung betrifft eine Viruslösung, die ein Detergens der allgemeinen Formel I enthält und der Stabilisierung der Viruslösung dient sowie deren Herstellung und Verwendung in heterogenen Immunoassays zum Nachweis von Antikörpern gegen Viren.

## Beschreibung

Die Erfindung betrifft eine Viruslösung, die ein Detergens der allgemeinen Formel I 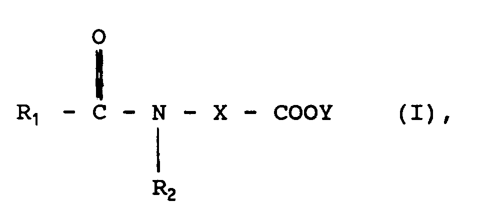
wobei
- R1:: ein hydrophober Rest,
- R2:: H oder ein Alkylrest mit 1 - 6 C-Atomen,
- X:: ein Alkylrest mit 1 - 6 C-Atomen und
- Y:: ein Alkali- oder Erdalkali-Ion ist,
enthält sowie deren Herstellung und Verwendung in einem heterogenen Immunoassay zum Nachweis von Antikörpern gegen Viren.

In der viralen Diagnostik werden zum Nachweis einer Infektion oft Antikörper bestimmt. Antikörper der IgM-Subklasse werden im Verlauf der Frühphase einer Infektion gebildet und persistieren einige Monate. Sie eignen sich daher zur Erfassung des akuten Krankheitsgeschehens. Neutralisierende Antikörper, meist vom IgG-Subtyp treten frühzeitig im Krankheitsverlauf auf und persistieren über viele Jahre. Ihr Nachweis ist von wesentlicher Aussage für Durchseuchungsstudien und zur Bewertung des Impfschutzes.

Zum Nachweis der Antikörper werden in zunehmendem Maße immunologische Methoden eingesetzt. Eine Vielzahl von Varianten sind bekannt, wobei sowohl kompetitive, immunoradiometrische und immunoenzymometrische als auch Sandwich-Assays bekannt sind. Eine Aufzählung verschiedener Varianten ist beispielsweise in einem Artikel von A. H. W. M. Schuurs und B. H. van Weemen in Dt. Ges. f. klin. Chemie e. V. -Mitteilungen 1/1979 angegeben. Unter anderem wird ein Testverfahren beschrieben, bei dem an eine Festphase ein gegen die Antikörper-Subklasse, der der zu bestimmende Antikörper entstammt, gerichteter Antikörper gebunden ist. In Gegenwart dieser Festphase wird die Probelösung, die den zu bestimmenden Antikörper enthält, mit dem für ihn spezifischen Antigen sowie einem ebenfalls mit dem Antigen bindefähigen markierten Antikörper inkubiert. Dabei konkurrieren der zu bestimmende Antikörper und der zugegebene markierte Antikörper um die Bindung an das Antigen. Die Immunkomplexe werden durch den festphasengebundenen Antikörper immobilisiert und nachgewiesen.

Falls das Antigen bei dieser Testvariante mehrere Epitope besitzt und so die gleichzeitige Bindung der zu bestimmenden Antikörper und der markierten Antikörper erlaubt, ohne daß diese beiden Antikörper miteinander konkurrieren beziehungsweise die Konkurrenz nur schwach ausgeprägt ist, kann sich ein Komplex aus dem an der Festphase gebundenen Antikörper, dem zu bestimmenden Antikörper, dem Antigen und dem markierten Antikörper ausbilden, der nachgewiesen werden kann.

Je nach Auswahl des markierten Antikörpers und des Antigens wird der zu bestimmende Antikörper somit durch ein indirektes (Konkurrenzbindungsverfahren) oder direktes Verfahren nachgewiesen.

In einer weiteren Testvariante ist an der Festphase ein Antikörper gegen das Virus-Antigen gebunden. Die Probenlösung, die den zu bestimmenden Antikörper enthält, wird mit dem für ihn spezifischen Virus-Antigen sowie einem ebenfalls mit dem Virus-Antigen bindefähigen markierten Antikörper inkubiert. Dabei konkurrieren der festphasengebundene Antikörper, der markierte Antikörper und der zu bestimmende Antikörper um die Bindung um das Antigen.

Der festphasengebundene Antikörper braucht nicht direkt an die Festphase gebunden zu sein sondern kann, falls gewünscht über ein spezifisch miteinander bindendes Paar gebunden werden, wie in der DE-A 3907651 offenbart. Als Antikörper wird dann ein Konjugat aus einem spezifisch bindefähigen Rezeptor und einer Substanz S verwendet, die ein Partner eines spezifisch miteinander bindenden Paares ist. Der andere Partner des spezifisch miteinander bindenden Paares ist an die Festphase gekoppelt.

In allen Testvarianten zum Nachweis der virus-spezifischen Antikörper wird ein Virus-Antigen zugesetzt. Dieses Virus-Antigen besteht aus Viruspartikeln oder aus Teilen davon.

Diese Viren oder Virusbestandteile werden in Form von Lyophylisaten oder konzentrierten Lösungen im Handel angeboten, die unmittelbar vor Gebrauch auf die gebrauchsfertige Konzentration verdünnt werden müssen. Im Zuge der besseren und einfacheren Handhabung ist es ein Bestreben, direkt gebrauchsfertige, bereits verdünnte Lösungen anzubieten. Verdünnte Viruslösungen sind jedoch sehr oft ungenügend lagerstabil und verursachen große, nicht tolerierbare Schwankungen des Meßsignals. Die genaue Ursache für die Instabilität ist nicht bekannt. Möglicherweise neigen einige Viren, insbesondere Viruspartikel, die hydrophobe Bestandteile besitzen, zur Aggregatbildung. Ein weiterer Nachteil dieser Aggregatbildung ist, daß hierdurch das Meßsignal erniedrigt wird. Um eine ausreichend empfindliche Messung zu erreichen, muß man im Ausgleich größere Mengen an Viren dem Test zusetzen.

Besonders ausgeprägt ist dieses Phänomen bei Hepatitis verursachenden Viren, insbesondere Hepatitis A-Viren sowie Herpes-Viren.

Aufgabe der vorliegenden Erfindung war es daher, diese Nachteile des Standes der Technik zu vermeiden und insbesondere eine Möglichkeit zu finden, Viren, und besonders solche, die zur Aggregatbildung neigen, in gebrauchsfertiger Lösung zu stabilisieren und hierdurch Schwankungen des Meßsignals zu vermeiden. Gleichzeitig soll die Empfindlichkeit beim Einsatz dieser Viruslösung in Immunoassays möglichst hoch sein.

Diese Aufgabe wird durch die in den Ansprüchen charakterisierte Erfindung gelöst. Insbesondere wird diese Aufgabe gelöst durch eine Lösung, die Viren oder Teile davon unter Zusatz eines Detergens der allgemeinen Formel I, 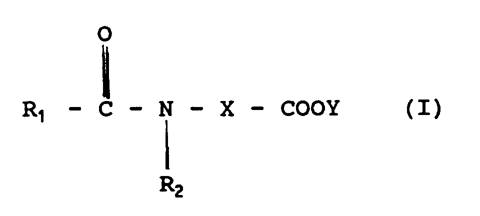
wobei
- R1:: ein hydrophober Rest,
- R2:: H oder ein Alkylrest mit 1 - 6 C-Atomen,
- X:: ein Alkylrest mit 1 - 6 C-Atomen und
- Y:: ein Alkali- oder Erdalkali-Ion ist,
enthält.

Ein Gegenstand der Erfindung ist weiterhin ein Verfahren zur Stabilisierung von Viruslösungen, die Herstellung der Viruslösung sowie deren Verwendung in einem heterogenen Immunoassay zur Bestimmung von Antikörpern gegen Viren.

Ein weiterer Gegenstand der Erfindung ist ein verbessertes Verfahren zur Bestimmung von Antikörpern gegen Viren nach dem Prinzip des heterogenen Immunoassays durch Inkubation der Probe mit der erfindungsgemäßen Lösung und mindestens zwei Rezeptoren R1 und R2, von denen R1 die Bindung an die feste Phase vermittelt und mit dem zu bestimmenden Antikörper oder mit den in der Lösung enthaltenden Viren spezifisch bindefähig ist und R2 ein Konjugat aus einem mit den in der Lösung enthaltenden Viren spezifisch bindefähigen Rezeptor und einer Markierung ist, Abtrennung des sich bildenden Komplexes aus der Lösung und Messen der Markierung in einer der Phasen.

Durch den Einsatz des Detergens der allgemeinen Formel I gelingt es überraschenderweise, die gebrauchsfertige Lösung zu stabilisieren. Gleichzeitig wird bei gleicher Einsatzmenge an Virusmaterial das Meßsignal erhöht und hierdurch die Sensitivität des Testes gesteigert. Die Steigerung der Sensitivität des Testes kann ebenfalls durch andere ionische Detergenzien bewirkt werden. Allerdings wird in keinem Fall, außer bei dem Zusatz des erfindungsgemäßen Detergens der allgemeinen Formel I eine Langzeitstabilisierung der gebrauchsfertigen Viruslösung beobachtet. Bei den anderen getesteten Detergenzien wurde anfänglich eine Steigerung des Signals im Test beobachtet. Bei einer Lagerung der gebrauchsfertigen Viruslösung, im allgemeinen bei 4 °C, tritt ein deutlicher, nicht zu kontrollierender Signalrückgang auf, was zu ungenauen Meßergebnissen führen kann.

Bei den Detergenzien, die erfindungsgemäß in der Viruslösung eingesetzt werden können, handelt es sich um die anionischen Detergenzien der allgemeinen Formel I

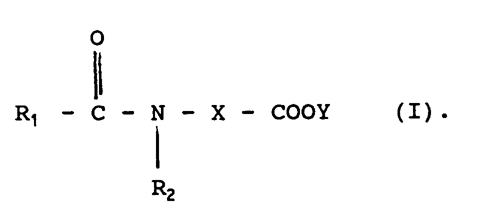

R1 bedeutet ein hydrophober Rest und stellt im allgemeinen einen gerad- oder verzweigtkettigen Alkylrest von 6 - 20 C-Atomen dar. R2 und X bedeuten gerad- oder verzweigtkettige Alkylreste mit 1 - 6 C-Atomen. Y bedeutet ein Kation. Alle Kationen, die nicht zur Unlöslichkeit der Verbindung führen, können verwendet werden. Insbesondere sind dies die Alkali- oder Erdalkali-Ionen, bevorzugt Natrium oder Kalium.

Besonders bevorzugt ist das Natriumsalz von N-Lauroylsarkosin (SLS) der allgemeinen Formel II. 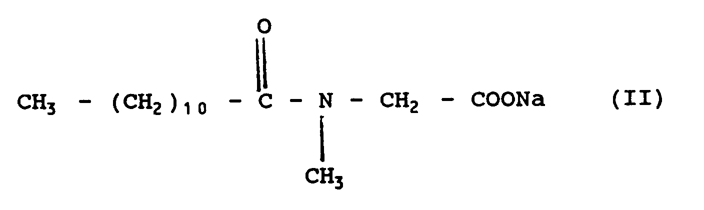
Zur Herstellung der Viruslösung wird ein aufgereinigter Virusstandard mit dem Detergens der Formel I versetzt und bis zur gebrauchsfertigen Konzentration verdünnt. Die Konzentration des Detergens in der gebrauchsfertigen Lösung beträgt 0,01 - 1 Gew. %, bevorzugt 0,04 - 0,4 und besonders bevorzugt 0,08 - 0,12 Gew. %. Neben dem Detergens und den Viruspartikeln oder Teilen davon kann die Lösung noch übliche Testzusätze wie beispielsweise Puffer, Albumin oder Konservierungsmittel, enthalten.

Als Viren werden besonders solche Viren eingesetzt, die sich durch eine starke Neigung zur Aggregation auszeichnen. Dies sind insbesondere Viren, die Hepatitis verursachen, wie Hepatitis A-, B-, C-, D- und E-Viren sowie Herpes-Viren. Ausgezeichnete Ergebnisse wurden mit Hepatitis A-Viren (HAV) erzielt. Neben den vollständigen Viruspartikeln können auch Teile davon, zum Beispiel die Virushülle, das Kapsid oder Kapsidproteine in der Lösung vorliegen. Die Konzentration der Viren oder der Virusbestandteile sind so gewählt, daß die Lösung direkt gebrauchsfertig ist, d. h. vor dem Gebrauch keine zusätzliche Verdünnung erforderlich ist. Die Konzentration wird also entsprechend den Erfordernissen der jeweiligen Nachweismethode angepaßt.

Nach Zugabe des Detergens wird die virushaltige Lösung inkubiert. Je nach der verwendeten Inkubationstemperatur von 2 - 35 °C wird die Stabilisierung nach ca. 15 Stunden bis 10 Tagen erreicht. Eine Inkubation für 26 - 30 Stunden bei 25 - 30 °C hat sich als besonders geeignet herausgestellt. Nach diesem Inkubationsintervall bleibt die gebrauchsfertige Viruslösung über einen Zeitraum von mehreren Monaten bei einer Lagerung bei 4 °c stabil.

Die erfindungsgemäße Viruslösung wird bei heterogenen Immunoassays zur Bestimmung von Antikörpern gegen Viren verwendet.

Ein weiterer Gegenstand der Erfindung sind daher solche heterogenen Immunoassays zur Bestimmung von Antikörpern gegen Viren, bei denen die erfindungsgemäße Viruslösung als ein wesentlicher Bestandteil eingesetzt wird. Bei dem erfindungsgemäßen Verfahren zur Bestimmung von Antikörpern gegen Viren nach dem Prinzip des heterogenen Immunoassays wird die Probe mit der lagerstabilen Viruslösung und mindestens zwei weiteren Rezeptoren R1 und R2 inkubiert. Der Rezeptor R1 vermittelt die Bindung an die feste Phase. Je nach dem, ob im Test klassenspezifische oder subklassenspezifische Antikörper, beispielsweise IgM, IgG₁, IgG₂ oder IgA, oder der Gesamtantikörpergehalt der Probe bestimmt werden sollen, ist der Rezeptor R1 mit dem zu bestimmenden Antikörper oder mit den in der lagerstabilen Lösung enthaltenen Viren spezifisch bindefähig.

Als Rezeptor R1 kann ebenfalls ein Konjugat verwendet werden, das aus einem mit dem zu bestimmenden Antikörper oder mit den in der lagerstabilen Lösung enthaltenen Viren spezifisch bindefähigen Rezeptor und einer spezifisch bindefähigen Substanz S besteht. Die spezifisch bindefähige Substanz S des Rezeptors R1 vermittelt die Bindung an die feste Phase. Die spezifisch bindefähige Substanz S ist dabei bevorzugt ein Partner eines spezifisch miteinander bindenden Paares. Derartige Paare sind dem Fachmann bekannt. Geeignet sind folgende Paare: Antigen-Antikörper; Hapten-Antikörper; Biotin-Avidin/Streptavidin; Protein-Antiprotein; Protein A-Immunglobulin; Hämoglobin-Haptoglobin oder Enzym-Substrat. Bevorzugt wird als S ein Hapten, wie beispielsweise Digoxin, p-Nitrophenol, Saponin, FITC und insbesondere Biotin verwendet. Die Bindung von S an den spezifisch mit dem zu bestimmenden Antikörper oder mit den in der erfindungsgemäßen Lösung enthaltenen Viren bindefähigen Rezeptor erfolgt in an sich bekannter Weise. Verfahren sind dem Fachmann bekannt.

Der zweite verwendete Rezeptor R2 ist ein Konjugat aus einem mit den in der erfindungsgemäßen Lösung enthaltenen Viren spezifisch bindefähigen Rezeptor und einer Markierung. Als Markierung kann ein Enzym, eine radioaktive Substanz, ein Isotop, eine fluoreszierende oder chemilumineszierende Substanz verwendet werden, deren Bestimmung nach einer dem Fachmann geläufigen Methode durchgeführt wird. Die Herstellung des Konjugats erfolgt in an sich bekannter Weise.

Bei dem Nachweis von klassenspezifischen oder subklassenspezifischen Antikörpern gegen das in der stabilen Lösung enthaltene Virus wird ein Rezeptor R1 eingesetzt, der mit dem zu bestimmenden Antikörper spezifisch bindefähig ist. Als Rezeptor R1 kommen Immunglobulin-klassen- oder subklassenspezifische mono- oder polyklonale Antikörper in Frage. Als spezifisch bindefähiger Rezeptor im Konjugat R2 wird ein Antikörper oder ein Antikörperfragment eingesetzt. Je nach dem, ob dieser Antikörper das selbe Epitop auf den Viren wie der zu bestimmende Antikörper erkennt oder nicht beziehungsweise ob auf den Viren ein oder mehrere gleiche Epitope vorhanden sind, konkurriert der Rezeptor R2 mit dem zu bestimmenden Antikörper um die Bindung an das Virusantigen oder bindet simultan an das Virusantigen. Bei der bevorzugten Testvariante, bei der keine oder nur eine geringfügige Konkurrenz der Probenantikörper und des Rezeptors R2 auftritt, bildet sich nach Inkubation der Testkomponenten ein Komplex aus wandgebundenem Rezeptor R1, Probenantikörper, Virusantigen und Rezeptor R2. Steigende Konzentrationen an Probenantikörper ergeben ein steigendes Meßsignal.

Bei dem Nachweis des Gesamtantikörpergehaltes der Probe wird ein Rezeptor R1 eingesetzt, der mit dem Virusantigen spezifisch bindefähig ist, ebenso wie der Rezeptor R2. Die für die Rezeptoren R1 und R2 in diesem Fall verwendeten spezifisch bindefähigen Rezeptoren sind Antikörper oder deren Fragmente, die mit den in der erfindungsgemäßen Lösung vorhandenen Viren bindefähig sind. Da diese spezifisch bindefähigen Rezeptoren mit dem zu bestimmenden Antikörper um die Bindung an das Virus konkurrieren sollen, ist es wesentlich, daß man hier Rezeptoren verwendet, die ähnlich wie der zu bestimmende Antikörper mit dem Virus reagieren, das heißt, eine ähnliche Bindungskapazität aufweisen. Daher werden als Rezeptoren für R1 und R2 entweder jeweils polyklonale Antikörper verwendet, die die selbe Bindefähigkeit wie der zu bestimmende Antikörper besitzen. Ebenso ist es möglich, monoklonale Antikörper zu verwenden, wenn gesichert ist, daß die von diesen monoklonalen Antikörpern erkannten Epitope am Virusantigen auch durch den zu bestimmenden Antikörper erkannt werden. Bei dieser Testvariante wird bei steigender Probenantikörperkonzentration ein abfallendes Meßsignal erzeugt.

Die Immobilisierung des sich bildenden Komplexes in beiden Testvarianten an die Festphase wird über den Rezeptor R1 vermittelt, der entweder direkt an der Festphase gebunden ist oder über die Substanz S an die Festphase gebunden werden kann. Bei der letzteren Variante werden die zu S komplementären Partner des spezifisch bindefähigen Paares an die feste Phase in an sich bekannter Weise gebunden. Als feste Phase sind sowohl Polymermaterialien als auch zellulosehaltige Materialien oder Glas geeignet. Als besonders geeignet haben sich Polystyrol, Polymetacrylat, Teflon, Polyamid, Copolymere aus Styrol und Acrylnitril, Glas und Zelluloseprodukte erwiesen. Die feste Phase kann in beliebiger Form vorliegen, zum Beispiel als Röhrchen, Microtiterplatten, Kugeln, Film, Pulver, Körnchen oder Faservlies.

Die spezifisch bindefähige Komponente kann in an sich bekannter Weise an die Festphase gebunden werden. Die Bindung kann dabei entweder direkt oder über einen Spacer oder ein Bindeprotein an die Festphase erfolgen. Als Verfahren ist beispielsweise das in der Patentanmeldung DE-A-3640412 beschriebene Verfahren zur Herstellung einer Festphasenmatrix geeignet.

Bei dem Nachweis von klassen- und subklassenspezifischen Antikörpern mit der bevorzugten Testvariante, bei der Probenantikörper und Rezeptor R2 beide gleichzeitig an das Virus binden und sich somit letztendlich ein Komplex aus Rezeptor R1, Probenantikörper, Virus und Rezeptor R2 ausbildet, wird bei der Testdurchführung zunächst der Rezeptor R2, der entweder direkt an die Festphase gebunden ist oder bevorzugt über die Substanz S an die Festphase gebunden wird, mit der Probe in dem Reaktionsgefäß inkubiert.

Es bildet sich zunächst ein festphasengebundener Komplex aus Rezeptor R2 und Probenantikörper. In einem zweiten Schritt, dem ebenfalls erforderlich ein Waschschritt vorgeschaltet sein kann, wird die erfingungsgemäße Viruslösung zugesetzt und erneut inkubiert. In einem dritten Arbeitsschritt wird der Rezeptor R2, der die Markierung trägt, hinzupipettiert. Zwischen dem zweiten und dritten Schritt kann, falls erforderlich, ebenfalls ein Waschschritt eingefügt werden.

Nach erneuter Inkubation wird die feste von der flüssigen Phase getrennt und die Markierung in einer der beiden Phasen in einem vierten Arbeitsschritt bestimmt.

Der Rezeptor R2 liegt als Konzentrat vor und muß vor Gebrauch auf die gewünschte Konzentration mit einem geeigneten Puffer der weitere übliche Zusatzstoffe wie Stabilisatoren der Markierung oder Konservierungsmittel enthalten kann, verdünnt werden.

Es hat sich als besonders bevorzugt erwiesen, den Rezeptor R2 vor Gebrauch anstatt mit einem besonderen Puffer mit der erfindungsgemäßen Lösung, die die Viren enthält, zu verdünnen. Die erfindungsgemäße Lösung kann in diesem Fall weitere Zusatzstoffe, die beispielsweise der Stabilisierung des Rezeptors R2 dienen, enthalten. Es bildet sich in dieser Lösung ein Komplex aus Rezeptor R2 und Virus. Dieser vorgebildete Immunkomplex kann direkt nach dem ersten Inkubationsschritt, bei dem Rezeptor und Probenantikörper inkubiert werden, eingesetzt werden.

In dem dritten Arbeitsschritt wird dann direkt die Markierung bestimmt. Hierdurch wird die Testführung, die bisher vier Arbeitsschritte erforderte, vereinfacht und beschleunigt.

Ein weiterer Gegenstand der vorliegenden Erfindung ist ein Testkit zur Bestimmung von Antikörpern, das die Rezeptoren R1 und R2, die lagerstabile Viruslösung sowie die feste Phase enthält, wobei R1 mit dem zu bestimmenden Antikörper oder mit dem in der lagerstabilen Lösung enthaltenen Viren spezifisch bindefähig ist und direkt an die Festphase gebunden ist oder ein Konjugat aus einem mit dem zu bestimmenden Antikörper oder mit den in der erfindungsgemäßen Lösung enthaltenen Viren spezifisch bindefähigen Rezeptor und einer spezifisch bindefähigen Substanz S und R2 ein Konjugat aus einem mit den in der Lösung enthaltenen Viren spezifisch bindefähigen Rezeptor und einer Markierung ist.

Als Festphase wird entweder eine Festphase verwendet, an der der Rezeptor R1 gebunden ist oder als bevorzugte Form eine Festphase an der eine mit S spezifisch bindefähige Komponente gebunden ist. Besonders bevorzugt erfolgt die Immobilisierung über das spezifisch bindende Paar Biotin-Avidin/Streptavidin.

Im Testkit liegen die Rezeptoren R1 und R2 sowie die erfindungsgmäße Lösung physikalisch getrennt voneinander vor. An die Festphase kann der Rezeptor R1 direkt gekoppelt sein. Im Falle, daß der Rezeptor R1 ein Konjugat aus S und einem spezifisch bindefähigen Rezeptor ist und an der Festphase eine mit S spezifisch bindefähige Komponente gebunden ist, liegt die Festphase von den Rezeptoren R1, R2 sowie der erfindungsgemäßen Lösung ebenfalls physikalisch getrennt vor.

Die Erfindung wird durch folgende Figuren und Beispiele erläutert:

### Beispiel 1:

### Herstellung einer lagerstabilen Hepatitis A-Viren-Lösung (HAV-Lösung)

Das Hepatitis A-Virus-Ausgangsmaterial wurde von der Firma Mediagnost, BR Deutschland bezogen. Die lagerstabile Lösung besitzt die folgende Zusammensetzung:
40 mM NA-Phosphat, Puffer pH 7,4
50 mM Dinatriumtartrat
0,1 % N-Lauroylsarcosin, Na-Salz
0,1 % Konservierungsmittel
0,2 % Rinderserumalbumin
Hepatitis A-Virus
Der Gehalt des Hepatitis A-Virus ist von Charge zu Charge schwankend und wird auf Signalbasis eingestellt, das heißt, daß im immunologischen Test annähernd das gleiche Signal erhalten wird.

Die Lösung wird nach der Zugabe des HAV-Antigens 28 Stunden bei 27,5 °C gerührt.

### Beispiel 2:

### Anti-HAV-IgM-Test

Die so hergestellte Lösung wurde in den kommerziell erhältlichen Enzymun-Test ® Anti-HAV-IgM der Firma Boehringer Mannheim GmbH eingesetzt. Folgende Lösungen wurden benutzt:

### Lösung 1 (Inkubationspuffer)

40 mM Phosphat-Puffer pH 7,0
ca. 1,5 µg/ml biotinylierter Anti-Human-IgM - monoklonaler Mausantikörper

### Lösung 2

Anti-HAV-monoklonaler Mausantikörper, POD-markiert
ca. 1 U/ml POD
Negative Kontrolle: Humanserum, das negativ für Anti-HAV ist
Positive Kontrolle: Humanes Anti-HAV-IgM in Humanserum
Die Lösung 2 wurde mit der HAV-Lösung, die unterschiedlich lang gelagert war, im Verhältnis von 100 : 1 (HAV-Lösung : Lösung 2) gemischt und vor der Testdurchführung mindestens eine Stunde inkubiert.

Im ersten Schritt wurden 10 µl Probe (negative Kontrolle beziehungsweise positive Kontrolle) mit 500 µl Inkubationspuffer in Streptavidin-beschichteten Polystyrolröhrchen 90 Minuten bei 25 °C inkubiert. Der biotinylierte Anti-Human-IgM-Antikörper bindet dabei das in der Probe enthaltene IgM und zusätzlich an die Streptavidin-beschichtete Tubewand.

Nach einmaligem Waschen werden im zweiten Schritt 500 µl des Gemisches aus HAV-Lösung und Lösung 2 in das Tube pipettiert und 90 Minuten bei 25 °C inkubiert. In diesem zweiten Schritt bindet der im Gemisch aus HAV-Lösung und Lösung 2 vorgebildete Detektionskomplex aus HAV-Antigen und Anti-HAV-Antikörper-POD-Konjugat spezifisch an das wandgebundene Anti-HAV-IgM der Probe.

Nach einem weiteren Waschschritt werden 500 µl Substrat-Chromogen-Lösung (100 mM Phosphat/Citrat-Puffer pH 4,4 mit 3,2 mM Natriumperborat werden zu gleichen Teilen mit 1,9 mM ABTS ® mindestens eine Stunde vor Testdurchführung gemischt) in das Tube pipettiert und nach einer 60-minütigen Inkubation bei 25 °C die gebildete Färbung bei 405 nm photometrisch bestimmt.

### Beispiel 3:

### Vergleich der Signalhöhe zwischen frisch angesetzter HAV-Lösung und der erfindungsgemäßen HAV-Lösung

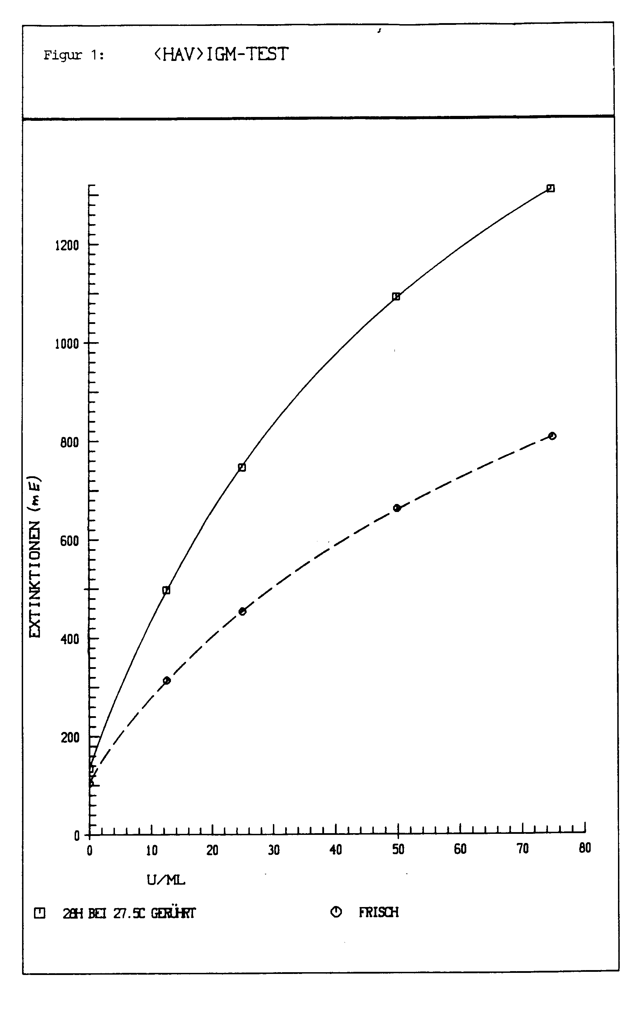

Die im Beispiel 1 hergestellte HAV-Lösung wurde mit einer frisch angesetzten HAV-Lösung, wobei die gleiche Pufferzusammensetzung benutzt wurde, im Anti-HAV-IgM-Test nach Beispiel 2 eingesetzt. Die Ergebnisse sind in Figur 1 graphisch dargestellt. Im Vergleich zur frisch angesetzten HAV-Lösung ergab sich bei der erfindungsgemäßen HAV-Lösung eine deutliche Steigerung der Signalhöhe (ca. 1,6-fach). Bei gleicher Einsatzmenge an HAV-Material wird also die Empfindlichkeit des Tests gesteigert. Falls die Empfindlichkeit des Testes nicht kritisch war, kann durch den Einsatz der erfindungsgemäßen HAV-Lösung die Einsatzmenge im Test reduziert werden, was im Falle des teuren HAV-Ausgangsmaterials sehr vorteilhaft ist.

### Beispiel 4

### Langzeitstabilität der HAV-Lösung

Die wie im Beispiel 1 hergestellte HAV-Lösung wurde bei 4 °C gelagert und anschließend die mit dieser Lösung zu erreichende Signalhöhe im Anti-HAV-Test entsprechend dem Beispiel 2 ermittelt. In der Tabelle 1 sind die Werte dargestellt. Der zu beobachtende langsame Drift zu etwas niedrigeren Signalen ist, wie durch Kontrollversuche belegt wurde, durch das bei der Messung zwangsläufig mitverwendete und ebenfalls bei 4 °C über die in der Tabelle angegebenen Zeiträume gelagerte flüssige POD-Konjugat (Lösung 2) bedingt.

**Tabelle 1**

| Lagerstabilität der HAV-Lösung | | |
|---|---|---|
| Lagerdauer bei 4 °C (Monate) | Extinktion (mE) bei 405 nm | |
| | negative Kontrolle | positive Kontrolle |
| 0 | 150 | 1015 |
| 1 | 142 | 1036 |
| 2 | 136 | 1120 |
| 3 | 141 | 989 |
| 4 | 125 | 922 |
| 5 | 147 | 907 |
| 6 | 128 | 872 |
| 9 | 132 | 785 |

### Beispiel 5

### Einfluß der Inkübationstemperatur bei der Herstellung

Die HAV-Lösung wurde, wie im Beispiel 1 beschrieben, hergestellt, mit dem Unterschied, daß die Lösung nach Zugabe des HAV-Antigens bei verschiedenen Temperaturen unterschiedlich lange inkubiert wurde. Anschließende wurde die Signalhöhe im Anti-HAV-IgM-Test entsprechend dem Beispiel 2 mit einer positiven Kontrolle als Probe ermittelt. Die gemessenen Extinktionen sind in Tabelle 2 dargestellt. Die Extinktion wird in allen Fällen von ca. 400 mE auf ca. 1000 mE gesteigert. Bei niedrigen Temperaturen von 4 °C dauert dieser Prozess ca. 7 Tage. Bei 25 und 30 °C reicht dagegen einen ca. eintägige Inkubation aus. Nach diesem Inkubationsintervall sind die so hergestellten HAV-Lösungen lagerstabil, das heißt, die Signalhöhe fällt durch die Lagerung nicht mehr auf den Anfangswert ab und bleibt, wie im vorhergehenden Beispiel gezeigt, über einen längeren Zeitraum hinweg konstant.

**Tabelle 2**

| Einfluß der Inkubationstemperatur bei der Herstellung | | | | | | | |
|---|---|---|---|---|---|---|---|
| Herstelltemperatur | Extinktion (mE) der positiven Kontrolle zu den angegebenen Zeiten | | | | | | |
| | 3 h | 1 d | 2 d | 3 d | 4 d | 7 d | 10 d |
| 4 °C | 371 | 553 | 645 | 780 | 853 | 1004 | 979 |
| 13 °C | 402 | 608 | 792 | 921 | 1065 | 1035 | 1067 |
| 18 °C | 418 | 712 | 931 | 990 | 1101 | 1037 | 1044 |
| 25 °C | 440 | 957 | 1022 | 1051 | 1098 | 1074 | 1049 |
| 30 °C | 475 | 982 | 1071 | 1104 | 1098 | 1107 | 1059 |

## Patentansprüche

1. Lösung enthaltend Viren oder Teile davon, dadurch gekennzeichnet, daß ein Detergens der allgemeinen Formel I 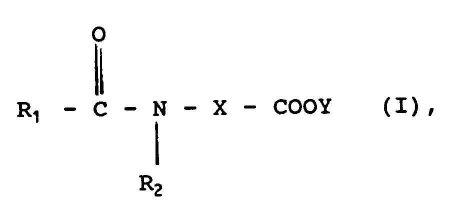 wobei
R₁ : ein hydrophober Rest ,
R₂: H oder ein Alkylrest mit 1 - 6 C-Atomen,
X: ein Alkylrest mit 1 - 6 C-Atomen und
Y: ein Alkali- oder Erdalkali-Ion ist,
enthalten ist.

2. Lösung gemäß Anspruch 1, dadurch gekennzeichnet, daß ein markierter, mit den Viren spezifisch bindefähiger Rezeptor R2 enthalten ist.

3. Herstellung der Lösung gemäß Anspruch 1, dadurch gekennzeichnet, daß man eine Lösung, die die Viren oder Teile hiervon enthält, mit einem Detergens der allgemeinen Formel I versetzt und die Mischung 18 Stunden bis 10 Tage bei 2 - 35 °C inkubiert.

4. Herstellung der Lösung gemäß Anspruch 3, dadurch gekennzeichnet, daß man die Mischung 26 - 30 Stunden bei 25 - 30 °C inkubiert.

5. Verfahren zur Bestimmung von Antikörpern gegen Viren nach dem Prinzip des heterogenen Immunoassays durch Inkubation der Probe mit der Lösung gemäß Anspruch 1 und mindestens 2 Rezeptoren R1 und R2, von denen R1 die Bindung an die feste Phase vermittelt und mit dem zu bestimmenden Antikörper oder mit den in der Lösung enthaltenen Viren spezifisch bindefähig ist und R2 ein Konjugat aus einem mit den in der Lösung enthaltenen Viren spezifisch bindefähigen Rezeptor und einer Markierung ist, Abtrennung des sich bildenden Komplexes aus der Lösung und Messung der Markierung in einer der Phasen.

6. Verfahren zur Bestimmung von Antikörpern einer bestimmten Immunglobin-Klasse oder -Subklasse gegen Viren nach dem Prinzip des heterogenen Immunoassays durch Inkubation der Probe mit der Lösung gemäß Anspruch 2 und mindestens einem Rezeptor R1, der in flüssiger Phase gelöst vorliegt und die Bindung an die feste Phase vermittelt und mit dem zu bestimmenden Antikörper spezifisch bindefähig ist, Abtrennung des sich bildenden Komplexes aus der Lösung und Messung der Markierung in einer der Phasen.

7. Verfahren gemäß Anspruch 6, dadurch gekennzeichnet, daß man die Probe in einem ersten Schritt mit dem Rezeptor R1 inkubiert und in einem zweiten Schritt nach einem gegebenenfalls notwendigen Waschschritt die Lösung gemäß Anspruch 2 hinzufügt und in einem dritten Schritt die Markierung mißt.

8. Verfahren gemäß einem der Ansprüche 5 - 7, dadurch gekennzeichnet, daß als Rezeptor R1 ein Konjugat aus einem mit dem zu bestimmenden Antikörper oder mit den in der Lösung enthaltenen Viren spezifisch bindefähigen Rezeptor und einer spezifisch bindefähigen Substanz S eingesetzt wird und man eine feste Phase verwendet, an der spezifisch mit S bindefähige Komponenten gebunden sind.

9. Verfahren zur Stabilisierung von Viren oder Teilen davon in einer gebrauchsfertigen verdünnten Lösung, dadurch gekennzeichnet, daß der Lösung ein Detergens der allgemeinen Formel I beigefügt wird.

10. Verwendung der Lösung gemäß einem der Ansprüche 1 oder 2 in einem heterogenen Immunoassay zur Bestimmung von Antikörpern gegen Viren.

11. Verwendung eines Detergens der allgemeinen Formel I zur Herstellung einer lagerstabilen Lösung, die Viren oder Teile davon enthält.

12. Testkit zur Bestimmung von Antikörpern nach dem Prinzip des heterogenen Immunoassays, dadurch gekennzeichnet, daß es die Lösung gemäß Anspruch 1, die Rezeptoren R1 und R2 sowie eine Festphase enthält, wobei R1 die Bindung an die feste Phase vermittelt und mit dem zu bestimmenden Antikörper oder mit den in der Lösung enthaltenen Viren spezifisch bindefähig ist und R2 ein Konjugat aus einem mit den in der Lösung enthaltenen Viren spezifisch bindefähigen Rezeptor und einer Markierung ist.

13. Testkit nach Anspruch 12, dadurch gekennzeichnet, daß es den Rezeptor R1 als ein Konjugat aus einem mit dem zu bestimmenden Antikörper oder mit den in der Lösung enthaltenen Viren bindefähigen Rezeptor und einer spezifisch bindefähigen Substanz S enthält.

14. Testkit nach Anspruch 13, dadurch gekennzeichnet, daß es den Rezeptor R1 mit Biotin als spezifisch bindefähige Substanz S und eine Festphase, an deren Oberfläche Avidin oder Streptavidin gebunden ist, enthält.
